# EUROPEAN PATENT APPLICATION

(11) **EP 4 210 069 A1**
(43) Date of publication of application: **12.07.2023**
(21) Application number: 22150845.0
(22) Date of filing: 11.01.2022
(51) Int. Cl.: G16H 30/40, G16H 50/70, A61B 5/055, G06N 3/02

(54) **SYNTHETIC CONTRAST-ENHANCED CT IMAGES**

(71) Applicant: Bayer Aktiengesellschaft, 51373 Leverkusen (DE); Universitätsklinikum Essen, 45147 Essen (DE)
(72) Inventor: The designation of the inventor has not yet been filed
(74) Representative: BIP Patents

(57) **Abstract**

The present invention relates to systems, methods and computer programs for training and using a machine learning model to generate synthetic contrast-enhanced computed tomography images with the help of magnetic resonance contrast agents.

## Description

### FIELD

Systems, methods, and computer programs disclosed herein relate to training and using a machine learning model to generate synthetic contrast-enhanced computed tomography images.

### BACKGROUND

Medical imaging is the technique and process of imaging the interior of a body for clinical analysis and medical intervention, as well as visual representation of the function of some organs or tissues (physiology). Medical imaging seeks to reveal internal structures hidden by the skin and bones, as well as to diagnose and treat diseases.

Many types of medical imaging use contrast agents to enhance the visualization of normal and abnormal structures. Examples include conventional angiography, fluoroscopy, computed tomography (CT), ultrasound, and magnetic resonance imaging (MRI).

With all these methods, use can be made of substances which facilitate the depiction or delimitation of certain structures in an examination object. Said substances are referred to as contrast agents.

From their pattern of spreading in the tissue, contrast agents can be roughly divided into the following categories: extracellular, intravascular and intracellular contrast agents.

Extracellular contrast agents refer to low-molecular-weight, water-soluble compounds which, after intravenous administration, spread in the blood vessels and in the interstitial space. After a certain, comparatively short period of circulation in the blood circulation system, they are excreted via the kidneys. The extracellular MRI contrast agents include, for example, the gadolinium chelates gadobutrol (Gadovist^{®}), gadoteridol (Prohance^{®}), gadoteric acid (Dotarem^{®}), gadopentetic acid (Magnevist^{®}) and gadodiamide (Omnican^{®}).

Intravascular contrast agents (also known as blood pool contrast agents) remain in the intravascular space much longer and are excreted more slowly than their extracellular counterparts, thus providing a longer time window for the imaging of blood vessels. Gadofosveset, for example, is a gadolinium-based intravascular MRI contrast agent. It has been used as a trisodium salt monohydrate form (Ablavar^{®}). It binds to serum albumin, resulting in the long residence time of the contrast agent in the bloodstream (half-life in blood about 17 hours).

Ferumoxytol is another example of an intravascular contrast agent. Ferumoxytol a colloidal iron-carbohydrate complex approved for parenteral treatment of iron deficiency in chronic kidney disease when oral therapy is not feasible. Ferumoxytol is administered as an intravenous injection. Ferumoxytol is commercially available as a solution for intravenous injection under the brand name Rienso^{®} or Ferahme^{®}. The iron-carbohydrate complex exhibits superparamagnetic properties and can therefore be used (off-label) for contrast enhancement in MRI examinations (see, e.g.: L.P. Smits et al.: Evaluation of ultrasmall superparamagnetic iron-oxide (USPIO) enhanced MRI with ferumoxytol to quantify arterial wall inflammation, Atherosclerosis 2017, 263: 211-218).

Intracellular contrast agents are capable of targeting specific tissues (see e.g. Y.-D. Xiao et al.: MRI contrast agents: Classification and application (Review), 2016, https://doi.org/10.3892/ijmm.2016.2744, in particular Table IV). Contrast agents based on gadoxetic acid are characterized by specific uptake by liver cells, the hepatocytes, by enrichment in the functional tissue (parenchyma) and by enhancement of the contrasts in healthy liver tissue.

Examples of liver-specific MR contrast agents are described in US 6,039,931A; they are commercially available under the trade names Primovist^{®} or Eovist^{®} for example. The contrast-enhancing effect of Primovist^{®}/Eovist^{®} is mediated by the stable gadolinium complex Gd-EOB-DTPA (gadolinium ethoxybenzyl diethylenetriaminepentaacetic acid). DTPA forms, with the paramagnetic gadolinium ion, a complex which has an extremely high thermodynamic stability. The ethoxybenzyl radical (EOB) is the mediator of the hepatobiliary uptake of the contrast agent.

Primovist^{®} can be used, *inter alia,* for the detection of tumors in the liver. Blood supply to the healthy liver tissue is primarily achieved via the portal vein (*vena portae*), whereas the liver artery (*arteria hepatica*) supplies most primary tumors. After intravenous injection of a bolus of contrast agent, it is accordingly possible to observe a time delay between the signal rise of the healthy liver parenchyma and of the tumor.

While there are numerous tissue-specific contrast agents for magnetic resonance imaging, comparable contrast agents for computed tomography are rare. For example, no equivalent liver-specific intracellular contrast agent has been authorized for CT imaging and is available on the market.

Advances in both imaging and machine learning have synergistically led to a rapid rise in the potential use of artificial intelligence in various medical imaging tasks, such as risk assessment, detection, diagnosis, prognosis, and therapy response.

Nowadays, machine learning is used not only for classification of images or detection of symptoms, but also for the generation of synthetic images. For example, WO2019/074938A1 discloses that machine learning is able to predict a synthesized full-dose contrast agent image from a low-dose contrast agent image and a pre-dose image.

WO2018/048507A1 discloses a method for generating synthetic CT images from original MRI images using a trained convolutional neural network.

MRI and CT each have advantages and disadvantages and can complement each other in medical imaging. It would be desirable to expand the possibilities of imaging tissues in the human body in order to broaden the tools available to practitioners in medical imaging and diagnostics.

### SUMMARY

This desire is addressed by the subject matter of the different embodiments as well as of the independent claims of the present disclosure. Preferred embodiments of the present disclosure are defined in the dependent claims and described in the present specification and/or depicted in the figures.

In a first aspect, the present disclosure provides a method of training a machine learning model to generate a synthetic contrast-enhanced CT image that represents at least a portion of an examination region within an examination object, the method comprising the steps of:
- receiving a training data set, the training data set comprising, for each object of a multitude of objects, input CT data, and target CT data,
   - wherein the input CT data comprise one or more representations of an examination region within the object during a CT examination after administration of a first dose of an MR contrast agent,
   - wherein the target CT data represent at least a portion of the examination region within the object during a CT examination after administration of a second dose of an MR contrast agent,
   - wherein the first dose of the MR contrast agent is different from the second dose of the MR contrast agent,
- training the machine learning model, thereby obtaining a trained machine learning mode, wherein the training comprises:
   ∘ inputting the input CT data into the machine learning model,
      wherein the machine learning model is configured to generate, at least partially on the basis of the input CT data and model parameters, predicted CT data,
   ∘ receiving from the machine learning model the predicted CT data,
   ∘ computing a loss value, the loss value quantifying the deviations between the predicted CT data and the target CT data,
   ∘ modifying one or more of the model parameters to minimize the loss value,
- outputting the trained machine learning model, and/or storing the machine learning model on a data storage, and/or providing the trained machine learning model for predictive purposes.

In another aspect, the present disclosure provides a computer system comprising:
a processor; and
a memory storing an application program configured to perform, when executed by the processor, an operation, the operation comprising:
   - receiving a training data set, the training data set comprising, for each object of a multitude of objects, input CT data, and target CT data,
      - wherein the input CT data comprise one or more representations of an examination region within the object during a CT examination after administration of a first dose of an MR contrast agent,
      - wherein the target CT data represent at least a portion of the examination region within the object during a CT examination after administration of a second dose of an MR contrast agent,
      - wherein the first dose of the MR contrast agent is different from the second dose of the MR contrast agent,
   - training the machine learning model, thereby obtaining a trained machine learning mode, wherein the training comprises:
      ∘ inputting the input CT data into the machine learning model, wherein the machine learning model is configured to generate, at least partially on the basis of the input CT data and model parameters, predicted CT data,
      ∘ receiving from the machine learning model the predicted CT data,
      ∘ computing a loss value, the loss value quantifying the deviations between the predicted CT data and the target CT data,
      ∘ modifying one or more of the model parameters to minimize the loss value,
   - outputting the trained machine learning model, and/or storing the machine learning model on a data storage, and/or providing the trained machine learning model for predictive purposes.

In another aspect, the present disclosure provides a non-transitory computer readable medium having stored thereon software instructions that, when executed by a processor of a computer system, cause the computer system to execute the following steps:
- receiving a training data set, the training data set comprising, for each object of a multitude of objects, input CT data, and target CT data,
   - wherein the input CT data comprise one or more representations of an examination region within the object during a CT examination after administration of a first dose of an MR contrast agent,
   - wherein the target CT data represent at least a portion of the examination region within the object during a CT examination after administration of a second dose of an MR contrast agent,
   - wherein the first dose of the MR contrast agent is different from the second dose of the MR contrast agent,
- training the machine learning model, thereby obtaining a trained machine learning mode, wherein the training comprises:
   ∘ inputting the input CT data into the machine learning model, wherein the machine learning model is configured to generate, at least partially on the basis of the input CT data and model parameters, predicted CT data,
   ∘ receiving from the machine learning model the predicted CT data,
   ∘ computing a loss value, the loss value quantifying the deviations between the predicted CT data and the target CT data,
   ∘ modifying one or more of the model parameters to minimize the loss value,
- outputting the trained machine learning model, and/or storing the machine learning model on a data storage, and/or providing the trained machine learning model for predictive purposes.

In another aspect, the present disclosure provides a kit, the kit comprising an MR contrast agent and a non-transitory computer-readable storage medium, the computer-readable storage medium having stored thereon software instructions that, when executed by a processor of a computer system, cause the computer system to execute the following steps:
- receiving a training data set, the training data set comprising, for each object of a multitude of objects, input CT data, and target CT data,
   - wherein the input CT data comprise one or more representations of an examination region within the object during a CT examination after administration of a first dose of the MR contrast agent,
   - wherein the target CT data represent at least a portion of the examination region within the object during a CT examination after administration of a second dose of the MR contrast agent,
   - wherein the first dose of the MR contrast agent is different from the second dose of the MR contrast agent,
- training the machine learning model, thereby obtaining a trained machine learning mode, wherein the training comprises:
   ∘ inputting the input CT data into the machine learning model, wherein the machine learning model is configured to generate, at least partially on the basis of the input CT data and model parameters, predicted CT data,
   ∘ receiving from the machine learning model the predicted CT data,
   ∘ computing a loss value, the loss value quantifying the deviations between the predicted CT data and the target CT data,
   ∘ modifying one or more of the model parameters to minimize the loss value,
- outputting the trained machine learning model, and/or storing the machine learning model on a data storage, and/or providing the trained machine learning model for predictive purposes.

Further aspects of the present disclosure are described hereinafter.

### DETAILED DESCRIPTION

The invention will be more particularly elucidated below, without distinguishing between the aspects of the disclosure (methods, computer systems, computer-readable storage media, kits). On the contrary, the following elucidations are intended to apply analogously to all the aspects, irrespective of in which context (methods, computer systems, computer-readable storage media, kits) they occur.

If steps are stated in an order in the present description or in the claims, this does not necessarily mean that the disclosure is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides solutions for generating synthetic contrast-enhanced computed tomography (CT) images. In particular, the present disclosure provides solutions for generating synthetic contrast-enhanced computed tomography (CT) images based on contrast-enhanced CT images which have been acquired using MR contrast agents.

In the context of the present invention, an "MR contrast agent" is any agent suitable for or capable of improving the visibility of an examination region within an examination object (e.g. internal body structures) by using magnetic resonance imaging (MRI) techniques, regardless of the fact that they may be suitable for use in or in combination with other techniques, such as CT. Preferably, an "MR contrast agent" means a contrast agent that has been approved by a competent authority such as the FDA or EMEA for use in magnetic resonance imaging.

A synthetic contrast-enhanced CT image according to the present invention represents an examination region of an examination object.

The "examination object" is usually a living being, preferably a mammal, very particularly preferably a human.

The "examination region" is a portion of the examination object, for example an organ or a portion of an organ, such as the liver, the heart, the lungs, the brain, the kidneys, lymph nodes and/or others. Preferably, the examination region is the liver or a portion of the liver of a mammal or the brain or a portion of the brain of a mammal (preferably a human). The examination region, also called image volume (field of view, FOV), is in particular a volume which is imaged in the magnetic resonance images. The examination region is typically defined by a radiologist, for example on an overview image (localizer). It is self-evident that the examination region can, alternatively or additionally, also be defined automatically, for example on the basis of a selected protocol.

The representation of the examination region can be a spatial representation and/or a frequency representation and/or any other representation. Usually, the representation of the examination region is a spatial representation, also referred to as image.

The term "image", as used herein, means a data structure that represents a spatial distribution of a physical signal (e.g., intensity of X-rays). The spatial distribution may be of any dimension, for example 2D, 3D, 4D or any higher dimension. The spatial distribution may be of any shape, for example forming a grid and thereby defining pixels, the grid being possibly irregular or regular. The representation of the spatial distribution of the physical signal can e.g. be a color or level of gray, such that the image may be a 2D or 3D RGB/grayscale image.

A "synthetic representation" is an artificially generated representation of an examination region of an examination object.

The term "synthetic" means that the representation is not the result of a (real) physical measurement, but of a calculated prediction. The synthetic contrast-enhanced representation may, for example, show the examination region as it would appear as a result of a CT examination after a high-dose (e.g., a dose higher than the standard dose) of an MR contrast agent has been applied - without this dose actually having been applied. The synthetic contrast-enhanced representation can be predicted on the basis of CT data, the CT data representing the examination region after the (real) application of a low-dose, i.e. an established dose of an MR contrast agent.

In other words: CT data representing the examination region after the application of a first dose of an MR contrast agent are used to predict a synthetic contrast-enhanced representation that represents the examination region after the application of a second dose of an MR contrast agent, wherein the second dose is different from, preferably higher than the first dose.

The prediction of the synthetic contrast-enhanced image is done, at least partially, on the basis of CT data. The CT data are a result of one or more (real) CT examinations of the examination object.

The CT data comprise one or more representations of the examination region within the examination object during a CT examination after administration of a first dose of the MR contrast agent. Preferably, the CT data comprise a plurality of representations of the examination region. The term "plurality" means more than one, e.g., 2, 3, 4, or 5, or even a higher number. Usually, each of the representations represents the examination region differently. In other words: in each representation, the examination region has a different appearance. It is for example possible, that the contrast distribution is different, and/or that the signal intensities of one or more tissue types are different. Additionally or alternatively, the distribution of the contrast agent within the examination region can be different. Additionally or alternatively, different CT data can represent different areas (portions) of the examination region, whereas the areas (portions) may partially overlap.

The target CT data represent at least a portion of the examination region within the examination object after administration of a second dose of an MR contrast agent. Thus, some methods of the present invention make use of a first dose and of a second dose of an MR contrast agent to provide CT data, i.e. input CT data and target CT data, respectively. It is preferred that the MR contrast agent employed to provide the input CT data and the target CT data, respectively, is the same MR contrast agent. It is conceivable, however, that the input CT data and the target CT data be provided using different MR contrast agents, as long as said MR contrast agents are compatible and suitable for the purposes of providing input and target CT data, respectively.

The first dose of an MR agent is preferably a dose which is equal to or less than the dose which is recommended by the manufacturer or distributor of the MR contrast agent and/or than the standard dose approved by an authority for an MR examination using the MR contrast agent. The recommended dose is usually the dose which is mentioned in the product label of the contrast agent and the dose approved by an authority (e.g. the US Food and Drug Administration (FDA) or the European Medicines Agency (EMA)) for an MR examination (also referred to as standard dose). Preferably, the first dose is equal to or less than a standard dose approved by an authority (e.g. the FDA or the EMA) for an MR examination. However, the first dose may also be higher than the standard dose. Thus, in another preferred embodiment, the first dose of an MR contrast agent is a dose that is required to obtain a defined appearance and/or capable of producing target CT data of sufficient quality, i.e. a representation of at least a portion of the examination region within the object of sufficient quality (e.g. a defined contrast enhancement) of the (first) examination region.

The first dose of an MR agent which allows the provision of input CT data is different than the second dose of an MR agent which allows the provision of target CT data. The first dose of an MR agent which allows the provision of input CT data is preferably lower than the second dose of an MR agent which allows the provision of target CT data. In other words, the second dose of an MR agent is higher than the first dose of an MR agent. Preferably, the second dose of an MR agent is capable of producing target CT data of sufficient quality, i.e. a representation of at least a portion of the examination region within the object of sufficient quality. To provide such target CT data, the second dose of an MR contrast agent may preferably be up to 100 times higher than the first dose of an MR contrast agent. More preferably, the second dose of an MR contrast agent is 10, 5 or 2 times higher than the first dose of an MR contrast agent.

Alternatively, and irrespective of the first dose used for the provision of input CT data, the second dose of an MR contrast agent is preferably higher, preferably up to 100 times higher, more preferably up to 10 times higher than the dose which is recommended by the manufacturer or distributor of the contrast agent and/or than the standard dose approved by an authority for an MR examination using the MR contrast agent.

The second dose of an MR agent which allows the provision of target CT data is preferably higher than the first dose of an MR agent which allows the provision of input CT data. Target CT data using a second dose of an MR contrast agent are typically collected from a multitude of examination objects. The term "multitude" preferably means more than ten, more preferably more than 100. However, given that the use of MR contrast agents may be associated with side effects, the provision of target CT data using a second dose of an MR contrast agent may or may not be achieved by collection from subjects other than test subjects. Depending on the second dose employed, it may be necessary and/or advantageous to obtain target CT data from animals and/or inanimate objects and/or non-living beings, such as corpses and/or cadavers. Alternatively, it may be necessary and/or advantageous to obtain target CT data from artificial objects which structural and mechanical features resemble those of an examination object, but which are not affected by any possible side effects derived from the use of high doses of an MR contrast agent. Thus, it is conceivable that the target CT data be acquired on an inanimate object. It is also conceivable that the target CT data are generated artificially, for example by an expert (e.g., a radiologist and/or a graphic designer) on a computer.

The MR contrast agent can be any MR contrast agent approved by an authority, such as the FDA or the EMA for MRI. By way of example, MR contrast agents typically used comprise gadolinium chelates like gadopentetate dimeglumine (trade name: Magnevist^{®} and others), gadobenate dimeglumine (trade name: Multihance^{®}), gadoteric acid (Dotarem^{®}, Dotagita^{®}, Cyclolux^{®}), gadodiamide (Omniscan^{®}), gadoteridol (ProHance^{®}), gadobutrol (Gadovist^{®}) and the stable gadolinium complex Gd-EOB-DTPA (gadolinium ethoxybenzyldiethylenetriaminepentaacetic acid) Primovist^{®}/Eovist^{®}.

In a preferred embodiment, the MR contrast agent is an intracellular contrast agent. More preferably, the MR contrast agent is an intracellular contrast agent distinguished by the fact that it is proportionately specifically taken up by liver cells, accumulates in the functional tissue (parenchyma) and enhances the contrasts in healthy liver tissue (e.g., a hepatobiliary MR contrast agent). More preferably, the MR contrast agent is a contrast agent based on gadoxetic acid such as the stable gadolinium complex Gd-EOB-DTPA (gadolinium ethoxybenzyldiethylenetriaminepentaacetic acid) Primovist^{®} and Eovist^{®}. Primovist^{®} is injected intravenously as a bolus, in a weight-adapted dose. The recommended dose is 0.025 mmol/kg body weight or 0.1 ml/kg body weight. In the context of the present disclosure, a dose corresponding to 100% of the second dose of the contrast agent signifies, for example in connection with Gd-EOB-DTPA disodium, that 0.025 mmol/kg body weight or 0.1 ml/kg body weight is administered to the examination object. Accordingly, "preferably less than 50% of the second dose" signifies that at most 0.0125 mmol/kg body weight or at most 0.05 ml/kg body weight is administered to the examination object.

In another preferred embodiment, the MR contrast agent is an intravascular contrast agent, such as Gadofosveset or Ferumoxytol or any other blood pool contrast agent.

The input and target CT data can be or comprise one or more images, i.e. spatial representation(s) of the examination region, the input and target CT data can be or comprise data representing the examination region in frequency space, and/or the input and target CT data can be one or more other representations of the examination region.

The input CT data can comprise one or more representations of the examination region without any contrast agent (e.g., before a contrast agent is administered).

The input CT data can comprise a plurality of representation of the examination region with different amounts of contrast agents applied.

The input CT data can comprise a plurality of representations of the examination region, each representation being generated at a different time point before and/or after administration of one or more doses of an MR contrast agent.

The input CT data can comprise, in addition to one or more representations after application of an MR contrast agents, one or more representations after application of a CT contrast agent.

The input CT data can comprise, in addition to one or more representations of the examination region, one or more representations of one or more other regions, e.g., one or more regions adjacent to the examination region. The other region(s) can be separated from the examination region, or can partially overlap with the examination region, or can be located within the examination region or can comprise the examination region. The degree of overlap can be, e.g., in the range of 0% to 50%.

The generation of synthetic contrast-enhanced representations in accordance with the present disclosure makes use of a trained machine learning model.

Such a "machine learning model", as described herein, may be understood as a computer implemented data processing architecture. The machine learning model can receive input data and provide output data based on that input data and the machine learning model, in particular the parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training to evaluate the machine learning model. For example, a loss function can include a metric of comparison of the output and the target. The loss function may be chosen in such a way that it rewards a wanted relation between output and target and/or penalizes an unwanted relation between an output and a target. Such a relation can be e.g. a similarity, or a dissimilarity, or another relation.

A loss function can be used to calculate a loss value for a given pair of output and target. The aim of the training process can be to modify (adjust) parameters of the machine learning model in order to reduce the loss value to a (defined) minimum.

A loss function may for example quantify the deviation between the output of the machine learning model for a given input and the target. If, for example, the output and the target are numbers, the loss function could be the difference between these numbers, or alternatively the absolute value of the difference. In this case, a high absolute value of the loss function can mean that a parameter of the model needs to undergo a strong change.

In the case of a scalar output, a loss function may be a difference metric such as an absolute value of a difference, a squared difference.

In the case of vector-valued outputs, for example, difference metrics between vectors such as the root mean square error, a cosine distance, a norm of the difference vector such as a Euclidean distance, a Chebyshev distance, an Lp-norm of a difference vector, a weighted norm or any other type of difference metric of two vectors can be chosen. These two vectors may for example be the desired output (target) and the actual output.

For training the machine learning model according to the present disclosure, a training data set is required, the training data set comprising, for each object of a multitude of objects, input CT data (e.g. first CT data, second CT data and optionally further data such as e.g. third CT data, fourth CT data, and so on, as described herein), and target CT data. The term "multitude" as it is used herein means an integer greater than 1, usually greater than 10, preferably greater than 100.

It should be mentioned that the training data set can be enlarged by augmentation techniques (see e.g. J. Nalepa et al.: Data Augmentation for Brain-Tumor Segmentation: A Review, Front. Comput. Neurosci., 11 December 2019 | https://doi.org/10.3389/fncom.2019.00083.

During training, input CT data (and optionally further data) are inputted into the machine learning model. The machine learning model is configured to generate, at least partially on the basis of the inputted input CT data, and on the basis of model parameters, predicted CT data.

The predicted CT data are compared with the target CT data. A loss value using a loss function can be computed, the loss value quantifying the deviations between the predicted CT data and the target CT data.

During training, model parameters are modified in order to reduce the loss values to a defined minimum.

Once the pre-defined minimum is reached and/or the predictions generated by machine learning model meet pre-defined criteria, the training can be finished.

A validation data set can be used to determine the accuracy of the trained machine learning model.

The trained machine learning model can be outputted and/or stored in a data storage and/or used for predictive purposes.

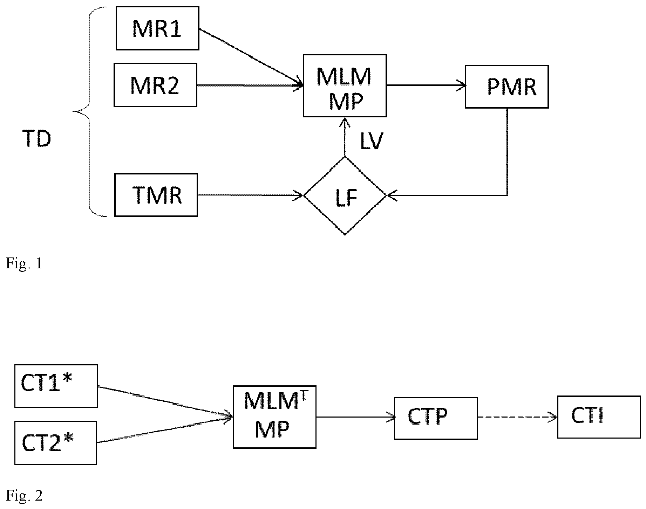

Fig. 1 shows schematically by way of example, the training of a machine learning model. The training is based on a training data set TD. The training data set TD comprises, for each object of a multitude of objects, first input CT data, second input CT data, and target CT data. In the example shown in Fig. 1, only one data set comprising first input CT data CT1, second input CT data CT2 and target CT data CTT is shown. First input CT data CT1 and second input CT data CT2 are inputted into the machine learning model MLM. The machine learning model MLM is configured to generate, at least partially on the basis of the inputted data and model parameters MP, predicted CT data CTP. The predicted CT data CTP are compared with the target CT data CTT. This is done by using a loss function LF, the loss function quantifying the deviations between the predicted CT data CTP and the target CT data CTT. For each pair of predicted CT data and the respective target CT data, a loss value LV is computed. During training the model parameters MP are modified in a way that reduces the loss values to a defined minimum. The aim of the training is to let the machine learning model MLM generate for each set of first input CT data and second input CT data predicted CT data which come as close to the corresponding target CT data as possible. Once the defined minimum is reached, the (now fully trained) machine learning model can be used to predict an output for new input data (input data which have not been used during training and for which the target is usually not (yet) known). First input CT data can, for example, comprise one or more representations of an examination region within the object during a CT examination before administration of a first dose of an MR contrast agent, and second input CT data can, for example, comprise one or more representations of the examination region within the object during the CT examination after administration of the first dose of the MR contrast agent.

Fig. 2 shows schematically by way of example how a trained machine learning model can be used for making predictions. The trained machine learning model MLM^{T} can be the machine learning model described with reference to Fig. 1. New input data comprising new first input CT data CT1* and new second input CT data CT2* are inputted into the trained machine learning model MLM^{T}. The trained machine learning model MLM^{T} is configured and trained to generate, at least partially on the basis of the new input data and the model parameters MD, predicted CT data CTP. First input CT data CT1* can, for example, comprise one or more representations of an examination region within the examination object during a CT examination before administration of a first dose of an MR contrast agent, and second input CT2* data can, for example, comprise one or more representations of the examination region within the examination object during the CT examination after administration of the first dose of the MR contrast agent.

The predicted CT data represents at least a portion of the examination region after application of a second dose of an MR contrast agent, the second dose being different from (preferably higher than) the first dose.

Depending on the nature of the input CT data, the predicted CT data can be a spatial representation of the examination region, or a representation of the examination region in frequency space, or another representation of the examination region. If for example, the input CT data comprise one or more images (i.e. spatial representations) of the examination region, then the predicted CT data preferably (but not necessarily) constitute an image of the examination region, too. If for example, the input CT data comprise one or more representations of the examination region in frequency space, or any other representation of the examination region, then the predicted CT data preferably (but not necessarily) constitute a representation in frequency space (or the other representation) of the examination, too.

In case the predicted CT data do not constitute an image (i.e. a spatial representation) or do not comprise an image, a further step may be necessary to transform the predicted CT data into an image; e.g. an inverse Fourier-transformation may be required to transform representations in frequency space into an image. This is reflected in Fig. 2 by the dashed arrow which indicates one or more optional transformation steps to transform the predicted CT data CTP into one or more synthetic contrast-enhanced image(s) CTI.

It should be noted that using representations of examination regions in frequency space may have advantages over using representations of examination regions in real space.

One advantage of the use of representations in frequency space of the examination region may be that contrast information is separated from detail information (e.g., fine structures). It is thus possible to concentrate, during a training procedure, on the contrast information to be learnt by the predictive machine learning model and to also concentrate, during a prediction procedure, on the contrast information that may be predicted by the trained machine learning model. Whereas contrast information in a representation in real space of an examination region is usually distributed over an entire representation (e.g., each image element intrinsically bears information about contrast), the contrast information in a representation in frequency space of an examination region is encoded in and around the center of the frequency space. Accordingly, the low frequencies in a representation in frequency space of an examination region are responsible for the contrast information, whereas the high frequencies contain detail information about fine structures. Using of representations in frequency space of the examination region may make it possible to separate the contrast information, to limit training and prediction to the contrast information, and to re-introduce information about the fine structures after a training procedure and/or a prediction procedure.

Further, representations in frequency space of the examination region may be more tolerant with respect to errors in co-registration during training, validation, and prediction procedures than representations in real space of the examination region. For example, if a representation in frequency space is superimposed with another representation in frequency space with less accuracy, the lack of accuracy has less influence than if representations in real space of the examination region are superimposed with less accuracy.

Thus, in a preferred embodiment of the present disclosure, input CT data, target CT data and predicted CT data comprise one or more representations in frequency space of the examination region (and optionally further regions).

The machine learning model according to the present disclosure can be or comprise one or more artificial neural networks.

An artificial neural network (ANN) is a biologically inspired computational model. An ANN usually comprises at least three layers of processing elements: a first layer with input neurons (nodes), a kth layer with at least one output neuron (node), and *k-*2 inner (hidden) layers, where *k* is an integer greater than 2.

In such a network, the input neurons serve to receive the input data. If the input data constitute or comprise an image (e.g. a spectrogram representation), there is usually one input neuron for each pixel/voxel of the input image; there can be additional input neurons for additional input data such as data about measurement conditions, data about the subject which generated the body action and/or the like. The output neurons serve to output the output data (result(s)).

The processing elements of the layers are interconnected in a predetermined pattern with predetermined connection weights therebetween. Each network node usually represents a (simple) calculation of the weighted sum of inputs from prior nodes and a non-linear output function. The combined calculation of the network nodes relates the inputs to the outputs.

When trained, the connection weights between the processing elements in the ANN contain information regarding the relationship between the input data and the output data which can be used to predict new output data from a new input data.

Each network node represents a calculation of the weighted sum of inputs from prior nodes and a non-linear output function. The combined calculation of the network nodes relates the inputs to the output(s).

Separate networks can be developed for each property measurement or groups of properties can be included in a single network. Preferably, different dimensions and/or modalities of patient data (and/or optionally additional data) are combined in a joint representation.

Training estimates network weights that allow the network to calculate (an) output value(s) close to the measured output value(s). A supervised training method can be used in which the output data is used to direct the training of the network weights. The network weights can be initialized with small random values or with the weights of a prior partially trained network. The training data inputs are applied to the network and the output values are calculated for each training sample. The network output values are compared to the measured output values. A backpropagation algorithm can be applied to correct the weight values in directions that reduce the error between measured and calculated outputs. The process is iterated until no further reduction in error can be made or until a predefined prediction accuracy has been reached.

A cross-validation method can be employed to split the data into training and validation data sets. The training data set is used in the backpropagation training of the network weights. The validation data set is used to verify that the trained network generalizes to make good predictions. The best network weight set can be taken as the one that best predicts the outputs of the training data. Similarly, varying the number of network hidden nodes and determining the network that performs best with the data sets optimizes the number of hidden nodes.

In a preferred embodiment of the present invention, the machine learning model is or comprises one or more convolutional neural networks (CNN).

A CNN is a class of deep neural networks, most commonly applied to analyzing visual imagery (such as spectrogram representations). A CNN comprises an input layer with input neurons, an output layer with at least one output neuron, as well as multiple hidden layers between the input layer and the output layer.

The hidden layers of a CNN typically consist of convolutional layers, ReLU (Rectified Linear Units) layer i.e. activation function, pooling layers, fully connected layers and normalization layers.

The nodes in the CNN input layer are organized into a set of "filters" (feature detectors), and the output of each set of filters is propagated to nodes in successive layers of the network. The computations for a CNN include applying the convolution mathematical operation to each filter to produce the output of that filter. Convolution is a specialized kind of mathematical operation performed by two functions to produce a third function that is a modified version of one of the two original functions. In convolutional network terminology, the first function to the convolution can be referred to as the input, while the second function can be referred to as the convolution kernel. The output may be referred to as the feature map. For example, the input to a convolution layer can be a multidimensional array of data that defines the various color components or grey scale values of an input image. The convolution kernel can be a multidimensional array of parameters, where the parameters are adapted by the training process for the neural network. More details about how to implement a convolutional neural network can be found in the literature (see e.g. Yu Han Liu: Feature Extraction and Image Recognition with Convolutional Neural Networks, 2018, J. Phys.: Conf. Ser. 1087 062032; H. H. Aghdam et al.: Guide to Convolutional Neural Networks, Springer 2017, ISBN: 978-3-319-57549-0; S. Khan et al.: Convolutional Neural Networks for Computer Vision, Morgan & Claypool Publishers, 2018, ISBN: 978-1-681-730219).

In a preferred embodiment of the present disclosure, the machine learning model is based on a specific kind of convolutional architecture called U-Net (see e.g. O. Ronneberger et al.: U-net: Convolutional networks for biomedical image segmentation, in: International Conference on Medical image computing and computer-assisted intervention, pp. 234-241, Springer, 2015, https://doi.org/10.1007/978-3-319-24574-4_28). The U-Net architecture consists of two main blocks, an encoding path and a decoding path. The encoding path uses convolutions, activation functions and pooling layers to extract image features, while the decoding path replaces the pooling layers with upsampling layers to project the extracted features back to pixel space, and finally recovers the image dimension at the end of the architecture. These are used in combination with activation functions and convolutions. Finally, the feature maps from the encoding paths can be concatenated to the feature maps in the decoding path in order to preserve fine details from the input data.

In another preferred embodiment, the machine learning model is or comprises a generative adversarial network (GAN), preferably a Pix2Pix GAN or a cycleGAN. A GAN is a class of machine learning frameworks in which two artificial neural networks contest with each other. A first neural networks is configured to generate a synthetic contrast-enhanced image, a second network is configured to distinguish synthetic contrast-enhanced images from real images. Both networks are trained simultaneously: the first network is trained to generate better synthetic images which the second network is not able to distinguish from real images, the second network is trained to distinguish increasingly good synthetic images from real images. It should be noted that the competing networks can be trained not only with images but also with other representations (such as representations in frequency space). It should be noted that artificial neural networks intended for unpaired image-to-image translations can also be used. Details about GANs can be found e.g. in M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887. The network should preferably be adapted to the image depth and resolution of the CT data because many networks only work with 8bit and 256x256 pixels resolution. A particularly preferred machine learning model is disclosed by J. Haubold et al.: Contrast agent dose reduction in computed tomography with deep learning using a conditional generative adversarial network, Eur Radiol. 2021, 31(8): 6087-6095, doi: 10.1007/s00330-021-07714-2.

The loss value is computed on the basis of the predicted CT data and the target CT data using a loss function. Examples of loss functions that can be used include L1 loss, L2 loss, structure similarity index measure (SSIM) or combination of the above to name a few. More details about loss functions may be found in the scientific literature (see e.g.: K. Janocha et al.: On Loss Functions for Deep Neural Networks in Classification, 2017, arXiv:1702.05659v1 [cs.LG]; H. Zhao et al.: Loss Functions for Image Restoration with Neural Networks, 2018, arXiv:1511.08861v3 [cs.CV]).

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g. digital signal processor (DSP), microcontrollers, field programmable gate array (FPGA), application specific integrated circuit (ASIC), etc.) and other electronic computing devices.

The term "process" as used herein is intended to include any type of computation or manipulation or transformation of data represented as physical, e.g. electronic, phenomena which may occur or reside e.g. within registers and/or memories of at least one computer or processor. The term processor includes a single processing unit or a plurality of distributed or remote such units.

Any suitable input device, such as but not limited to a keyboard, a mouse, a microphone and/or a camera sensor, may be used to generate or otherwise provide information received by the system and methods shown and described herein. Any suitable output device or display, such as but not limited to a computer screen (monitor) and/or printer may be used to display or output information generated by the system and methods shown and described herein. Any suitable processor/s, such as bot not limited to a CPU, DSP, FPGA and/or ASIC, may be employed to compute or generate information as described herein and/or to perform functionalities described herein. Any suitable computerized data storage, such as but not limited to optical disks, CDROMs, DVDs, BluRays, magnetic-optical discs or other discs; RAMs, ROMs, EPROMs, EEPROMs, magnetic or optical or other cards, may be used to store information received by or generated by the systems shown and described herein. Functionalities shown and described herein may be divided between a server computer and a plurality of client computers. These or any other computerized components shown and described herein may communicate between themselves via a suitable computer network.

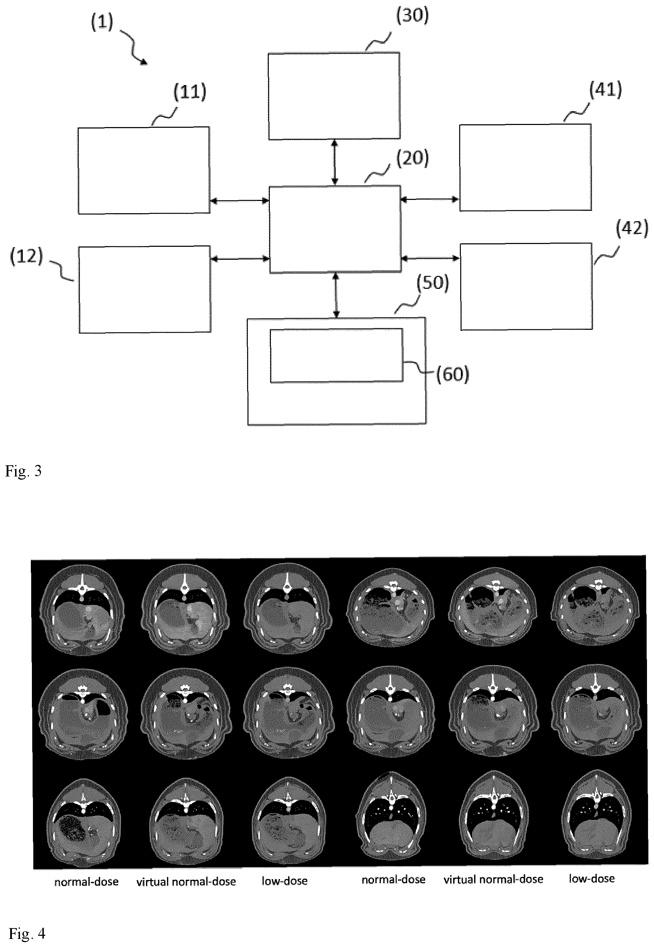

Fig. 3 illustrates a computer system (10) according to some example implementations of the present invention in more detail. Generally, a computer system of exemplary implementations of the present disclosure may be referred to as a computer and may comprise, include, or be embodied in one or more fixed or portable electronic devices. The computer may include one or more of each of a number of components such as, for example, processing unit (11) connected to a memory (15) (e.g., storage device).

The processing unit (11) may be composed of one or more processors alone or in combination with one or more memories. The processing unit is generally any piece of computer hardware that is capable of processing information such as, for example, data (incl. digital images), computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit (11) may be configured to execute computer programs, which may be stored onboard the processing unit or otherwise stored in the memory (15) of the same or another computer.

The processing unit (11) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (15) is generally any piece of computer hardware that is capable of storing information such as, for example, data, computer programs (e.g., computer-readable program code (16)) and/or other suitable information either on a temporary basis and/or a permanent basis. The memory may include volatile and/or non-volatile memory and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information and is distinguishable from computer-readable transmission media such as electronic transitory signals capable of carrying information from one location to another. Computer-readable medium as described herein may generally refer to a computer-readable storage medium or computer-readable transmission medium.

In addition to the memory (15), the processing unit (11) may also be connected to one or more interfaces (12, 13, 14, 17, 18) for displaying, transmitting and/or receiving information. The interfaces may include one or more communications interfaces (17, 18) and/or one or more user interfaces (12, 13, 14). The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) to connect to a network, such as using technologies such as cellular telephone, Wi-Fi, satellite, cable, digital subscriber line (DSL), fiber optics and the like. In some examples, the communications interface(s) may include one or more short-range communications interfaces configured to connect devices using short-range communications technologies such as NFC, RFID, Bluetooth, Bluetooth LE, ZigBee, infrared (e.g., IrDA) or the like.

The user interfaces (12, 13,14) may include a display (14). The display (14) may be configured to present or otherwise display information to a user, suitable examples of which include a liquid crystal display (LCD), light-emitting diode display (LED), plasma display panel (PDP) or the like. The user input interface(s) (12, 13) may be wired or wireless, and may be configured to receive information from a user into the computer system (10), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

### Example

In the following, an example implementation for virtual contrast enhancement in CT and for the use of MR and more specifically hepatobiliary contrast agent is described. The network was trained to convert image X2 with a contrast agent dose reduced by 71.4 % (100mgl/kg, Iopromid 300) to generate image Y with normal contrast agent dose (350mgl/kg, Iopromid 300). For this purpose, image X2, as well as image X1 above it and image X3 below it were given to the network as input. The images X2 and Y were located at the same position in the coordinate system. In the example implementation, image X2 was acquired with a dose of 100mgl/kg (Iopromid 300) and Y with a dose of 350mgl/kg (Iopromid 300). Furthermore, the datasets were co-registered and a virtual increase in the size of the collective was performed using data augmentation with the following parameters: Resize: 224; Crop Size: 224; Rotate: -15, 15; Horizontal Flip. A cycleGAN was used as a machine learning model with the following parameters: Cycle consistency Loss; Upsampling: transpose; Batch Size: 4; lr: 0.0002; lr_decay: linear; Epochs: 100 without decay + 100 with decay; net_generator: resnet9_blocks; net_discriminator: 70x70 PatchGan; normalization_type: instance normalization.

As a dataset, 20 healthy Göttingen minipigs underwent reduced-dose (100mgl/kg, Iopromid 300) and standard-dose (350mgl/kg, Iopromid 300) CT on three separate occasions, including an early arterial, late arterial, portal venous, and venous contrast phase. One animal had to be excluded due to incomplete examinations. Three were randomly selected and retained for validation (18 examinations). Subsequently, the machine learning model was trained to convert low-dose to normal-dose examinations with the remaining 16 animals (96 examinations). The efficiency of the network was validated quantitatively by measuring the contrast-to-noise ratio (CNR) and qualitatively via a Visual Turing Test (VTT). For CNR measurement, regions-of-interest (ROIs) were placed in the autochthonous back muscle to measure noise and the abdominal aorta, portal vein, and liver parenchyma to measure contrast. Subsequently, CNR was calculated by subtracting the attenuation of the autochthonous back muscles from the attenuation in the respective target structure divided by the standard deviation of the autochthonous back muscles. In addition, for qualitative evaluation in a Visual Turing Test (VTT), the data from the standard-dose and the virtual standard-dose examination were presented to three radiology consultants, who had to decide whether they would have classified both data sets as pathologically consistent and which images were from the standard-dose examination. They were blinded for the type of examination (standard-dose vs. virtual standard-dose) and furthermore, the side on which the standard dose images were shown was randomly changed from case to case.

Regarding the quantitative evaluation, the CNR increased significantly (P<0.0001) in all contrast phases and did not differ significantly from standard-dose examinations (1.4±1.0 low-dose, 5.0±4.8 virtual standard-dose, 5.1±5.7 standard-dose). For qualitative evaluation the three consultant radiologists reported on average in the VTT that the standard-dose and virtual standard-dose were pathological consistent in 93% of the examinations. On average, the reviewers were able to identify the standard-dose sequence as such in 80% of the cases. Example results of this implementation are shown in figure 4.

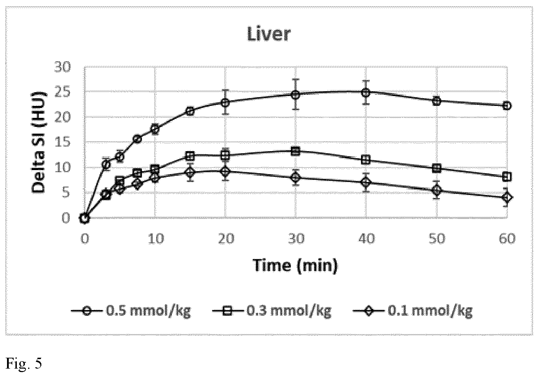

To implement hepatobiliary contrast agent on CT, a higher dose is required than recommended by the manufacturer for use on MRI. In a preliminary test in three rabbits, we observed an increase in the density of the liver parenchyma in the hepatobiliary contrast phase by approximately 25 HU at a dose increased by a factor of 20 (0.5 mmol/kg, Dinatriumgadoxetat) on CT. Measurements of the attenuation of liver parenchyma in the hepatobiliary contrast phase of our test collective are shown in figure 5.

In order to use a hepatobiliary a MRI contrast media for liver enhancement in CT the dose can be higher than the approved MRI dose, for example 0.1 mmol/kg. A respective collective with standard dose (0.1 mmol/kg) and 5-fold increased dose (0.5 mmol/kg) must be provided to the network. Considering that our method has been successfully employed to variety of dynamic contrast phases on CT, representing a similar magnitude of dose reduction, it is reasonable to assume that the principle can be applied to MR contrast agent and especially to hepatobiliary contrast agent. For this purpose, a sufficient number of CT examinations (preferably in an amount similar to our example implementation) with a dose (0.1 mmol/kg) and an dose increased by a factor of 5 (0.5 mmol/kg) need to be acquired. Thereafter, co-registration and data augmentation should be performed with the following parameters: Resize: 224; Crop Size: 224; Rotate: -15, 15; Horizontal Flip. Subsequently, a machine learning model preferably a GAN more preferably a cycleGAN can be trained with the parameter settings and input described by us in our example implementation: Cycle consistency Loss; Upsampling: transpose; Batch Size: 4; lr: 0.0002; lr_decay: linear; Epochs: 100 without decay + 100 with decay; net_generator: resnet9_blocks; net_discriminator: 70x70 PatchGan; normalization_type: instance normalization. Finally, the trained machine learning model can be used for predictions.

## Claims

1. A method comprising the steps:
- receiving a training data set, the training data set comprising, for each object of a multitude of objects, input CT data, and target CT data,
- wherein the input CT data comprise one or more representations of an examination region within the object during a CT examination after administration of a first dose of an MR contrast agent,
- wherein the target CT data represent at least a portion of the examination region within the object during a CT examination after administration of a second dose of an MR contrast agent,
- wherein the first dose of the MR contrast agent is different from the second dose of the MR contrast agent,
- training the machine learning model, thereby obtaining a trained machine learning mode, wherein the training comprises:
∘ inputting the input CT data into the machine learning model, wherein the machine learning model is configured to generate, at least partially on the basis of the input CT data and model parameters, predicted CT data,
∘ receiving from the machine learning model the predicted CT data,
∘ computing a loss value, the loss value quantifying the deviations between the predicted CT data and the target CT data,
∘ modifying one or more of the model parameters to minimize the loss value,
- outputting the trained machine learning model, and/or storing the machine learning model on a data storage, and/or providing the trained machine learning model for predictive purposes.

2. The method according to claim 1, wherein the examination region within the object is the liver.

3. The method according to any one of claims 1 or 2, wherein the MR contrast agent is an intracellular contrast agent, preferably wherein the MR contrast agent is based on gadoxetic acid or a gadoxetic acid derivative, more preferably wherein the MR contrast agent is Primovist^{®} or Eovist^{®}.

4. The method according to any one of claims 1 or 2, wherein the MR contrast agent is an intravascular contrast agent.

5. The method according to any one of claims 1 to 4, wherein the target CT data is acquired on an inanimate object.

6. The method according to any one of claims 1 to 5, wherein the first dose of the MR contrast agent is lower than the second dose of the MR contrast agent,

7. The method according to any one of claims 1 to 6, wherein the first dose of the MR contrast agent is a dose which is equal to or less than the dose which is recommended by the manufacturer or distributor of the contrast agent and/or equal to or less than a standard dose approved by an authority for an MR examination using the MR contrast agent.

8. The method according to any one of claims 1 to 7, wherein the second dose of the MR contrast agent is a dose which is up to 100 times, preferably up to 10 times higher than the dose which is recommended by the manufacturer or distributor of the contrast agent and/or than the standard dose approved by an authority for an MR examination using the MR contrast agent.

9. The method according to any one of claims 1 to 8,
- wherein the first dose of the MR contrast agent is a dose which is equal to or less than the dose which is recommended by the manufacturer or distributor of the contrast agent and/or equal to or less than a standard dose approved by an authority for an MR examination using the MR contrast agent, and
- wherein the second dose of the MR contrast agent is a dose which is up to 100 times, preferably up to 10 times higher than the dose which is recommended by the manufacturer or distributor of the contrast agent and/or than the standard dose approved by an authority for an MR examination using the MR contrast agent.

10. The method according to any one of claims 1 to 9, further comprising the steps
- receiving new input CT data, wherein the input CT data comprise one or more representations of the examination region within an examination object during a CT examination after administration of the first dose of the MR contrast agent,
- inputting the new input CT data into the trained machine learning model,
- receiving, from the trained machine learning model, predicted CT data, wherein the predicted CT data comprise a representation of the examination region of the examination object after administration of the second dose of the MR contrast agent,
- outputting the representation of the examination region of the examination object after administration of the second dose of the MR contrast agent.

11. A computer system comprising:
a processor; and
a memory storing an application program configured to perform, when executed by the processor, an operation, the operation comprising:
- receiving a training data set, the training data set comprising, for each object of a multitude of objects, input CT data, and target CT data,
- wherein the input CT data comprise one or more representations of an examination region within the object during a CT examination after administration of a first dose of an MR contrast agent,
- wherein the target CT data represent at least a portion of the examination region within the object during a CT examination after administration of a second dose of an MR contrast agent,
- wherein the first dose of the MR contrast agent is different from the second dose of the MR contrast agent,
- training the machine learning model, thereby obtaining a trained machine learning mode, wherein the training comprises:
∘ inputting the input CT data into the machine learning model, wherein the machine learning model is configured to generate, at least partially on the basis of the input CT data and model parameters, predicted CT data,
∘ receiving from the machine learning model the predicted CT data,
∘ computing a loss value, the loss value quantifying the deviations between the predicted CT data and the target CT data,
∘ modifying one or more of the model parameters to minimize the loss value,
- outputting the trained machine learning model, and/or storing the machine learning model on a data storage, and/or providing the trained machine learning model for predictive purposes.

12. A non-transitory computer readable medium having stored thereon software instructions that, when executed by a processor of a computer system, cause the computer system to execute the following steps:
- receiving a training data set, the training data set comprising, for each object of a multitude of objects, input CT data, and target CT data,
- wherein the input CT data comprise one or more representations of an examination region within the object during a CT examination after administration of a first dose of an MR contrast agent,
- wherein the target CT data represent at least a portion of the examination region within the object during a CT examination after administration of a second dose of an MR contrast agent,
- wherein the first dose of the MR contrast agent is different from the second dose of the MR contrast agent,
- training the machine learning model, thereby obtaining a trained machine learning mode, wherein the training comprises:
∘ inputting the input CT data into the machine learning model, wherein the machine learning model is configured to generate, at least partially on the basis of the input CT data and model parameters, predicted CT data,
∘ receiving from the machine learning model the predicted CT data,
∘ computing a loss value, the loss value quantifying the deviations between the predicted CT data and the target CT data,
∘ modifying one or more of the model parameters to minimize the loss value,
- outputting the trained machine learning model, and/or storing the machine learning model on a data storage, and/or providing the trained machine learning model for predictive purposes.

13. Kit comprising a contrast agent and a non-transitory computer-readable storage medium according to claim 12.

14. Kit according to claim 13, wherein the MR contrast agent is an intracellular contrast agent, preferably wherein the MR contrast agent is based on gadoxetic acid or a gadoxetic acid derivative, more preferably wherein the MR contrast agent is a substance or a substance mixture comprising gadoxetic acid or a gadoxetic acid salt as contrast-enhancing active substance, preferably the disodium salt of gadoxetic acid.

15. Kit according to claim 13, wherein the MR contrast agent is an intravascular contrast agent.
